# EUROPEAN PATENT SPECIFICATION

(11) **EP 3 347 069 B1**
(45) Date of publication and mention of the grant of the patent: **25.03.2020**
(21) Application number: 16844808.2
(22) Date of filing: 09.09.2016
(51) Int. Cl.: A61M 1/02, A61M 1/10

(54) **BLOOD MIXER FOR A BLOOD COLLECTING BAG**
BLUTMISCHER FÜR EINEN BLUTSAMMELBEUTEL
MÉLANGEUR DE SANG POUR UN SAC DE COLLECTE DE SANG

(30) Priority: 10.09.2015 SE 1551163
(43) Date of publication of application: 18.07.2018
(73) Proprietor: Conroy Medical AB, 194 63 Upplands Väsby (SE)
(72) Inventor: JANSSON, Per, 193 41 Sigtuna (SE); LUNDMAN, Nicklas, 113 20 Stockholm (SE)
(74) Representative: Brann AB
(86) International application number: PCT/SE2016/050844
(87) International publication number: WO 2017/044040

(56) References cited:
- EP-A2- 0 400 518
- WO-A1-98/48866
- WO-A1-2015/011473
- JP-A- 2012 115 613
- US-A- 4 385 630
- US-A- 4 923 449
- US-A- 5 098 372
- US-A- 5 147 330
- US-A- 5 690 815
- US-A- 6 033 561
- US-A- 6 106 509
- US-A1- 2003 055 396
- US-A1- 2003 146 170
- US-A1- 2012 120 384
- US-A1- 2012 269 679
- US-A1- 2015 157 780
- US-A1- 2015 238 672
- US-B1- 6 402 702
- US-B1- 7 608 043

## Description

### Technical field

The present invention relates to blood collection systems in general, and in particular to a mixer for a blood bag in which blood is collected from a donor.

### Background of the Invention

The task of taking blood from a donor requires many manual operations on the part of the person taking the blood. The blood collecting bags which are normally used, and also the hose connected to the bags and intended to be fitted to a cannula are prefilled with an anticoagulant.

In order to ascertain adequate mixing of blood and antigoagulant there are provided mixers provided with so called cradles in which the blood bag is placed.

Various mixers of this kind are known to the art. For instance, mixers are known which include a bag-carrying surface which is caused to rock forwards and backwards with the aid of a crank device which rotates about an axle which extends perpendicularly to the bag-carrying surface. It is also known to provide such mixer with a weight-measuring device which functions to measure the amount of blood that has flowed into the bag. It is also known to provide such mixers with means which function to produce an alarm signal when blood flows too quickly or too slowly into the bag.

Such a product is described in the granted US patent 5,147,330. It relates to a mixer (in the patent the entire device is referred to as a cradle) of the kind which includes a bag-carrying surface which is intended to be rocked reciprocatingly about an axle which extends perpendicularly to said surface, by means of a crank device or the like, said mixer being provided with a weight measuring device which functions to measure the quantity of blood that has flown into the blood bag. The mixer is also provided with a clamping device which functions to squeeze said hose so as to prevent blood from flowing therein, said clamping device being electromagnetically operable, and wherein the mixer is provided with a control circuit which functions to at least control said clamping device.

US 2012/010553 (A1) (Carlson et al) a blood collection system that exhibits features such as a support structure, a tray (describe as a "scale", but functions as a tray for a bag), a pump inherently disclosed since there must be some kind of pump in order to extract blood from the donor and a tilting mechanism ("blood agitating system").

### Summary of the Invention

An object of the present apparatus disclosed herein is to provide a blood mixer that will enable an operator to position the mixer at a convenient elevated position higher than the patient, and which ascertains that no blood will flow back from the collecting bag to the patient.

This object is achieved with a blood mixer as defined in claim 1.

It comprises a support structure supporting the components of the mixer, a tubing fastening and guiding arrangement, configured to secure a blood collecting tubing in the mixer, a weigh bowl adapted to house a blood collecting bag having a blood supply tube attached thereto, and configured to measure the weight of the collected blood in the blood collecting bag and a pump configured to draw blood from the donor to a blood collecting bag through said tubing. In particular the pump is configured to exert a kneading action on the blood supply tubing by means of at least two kneading elements arranged to alternatingly displace the contents of the tube in the direction towards the bag, over one segment of the tubing each, thereby preventing back flow inside the tubing. There is preferably also provided a cooling arrangement configured to cool the blood in the blood collecting bag.

### Brief description of the drawings

The detailed description given hereinafter is to be read with reference to the accompanying drawings which are given by way of illustration only, and thus not to be considered limiting on the present invention, and wherein
Fig. 1 illustrates a prior art mixer 1;
Fig. 2 shows the prior art device of Fig. 1 in a side view;
Fig. 3 is a schematic illustration of a complete blood collecting system including a mixer; and
Fig. 4 schematically illustrates a sensor unit.

### Detailed Description of Preferred Embodiments

For the purposes of this application the term "blood mixer" shall be taken to mean an apparatus comprising at least one movable part adapted to receive a blood colleting bag. The movement is preferably a tilting action, either a simple rocking or a multiaxis movement, so as to ascertain proper mixing of the contents of the bag, i.e. collected blood and any agents provide in the bag, such as anticoagulants. The entire apparatus could in principle be moveable/tiltable.

In particular the part adapted for receiving the blood collecting bag is referred to as a "bowl" or "tray". If a weighing function is integrated in the bowl it is referred to as a "weighing bowl" or "weighing tray".

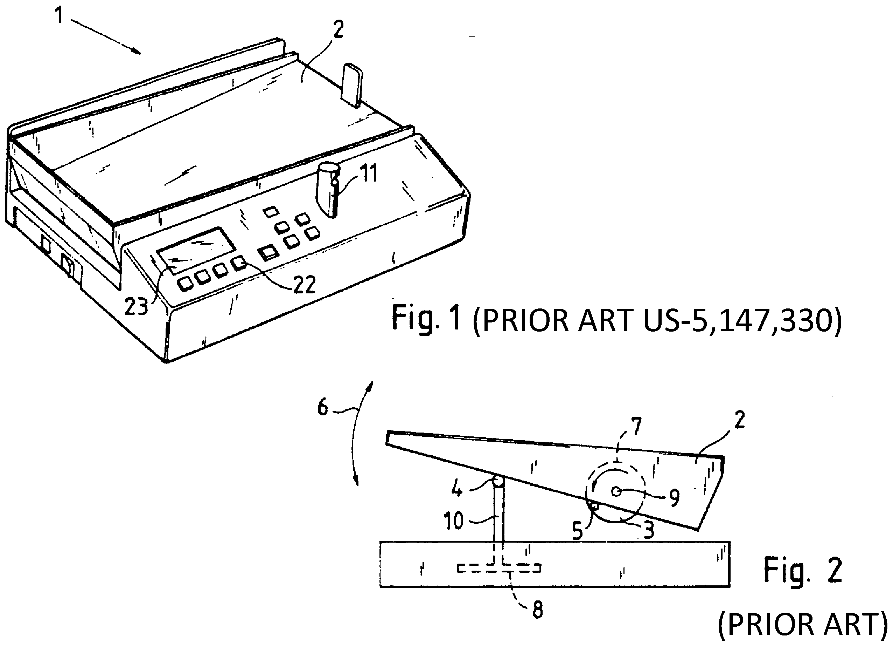

Figs. 1 and 2 schematically illustrates a prior art blood mixer device/system 1 for collecting blood from a patient. This mixer is intended for use in blood donating processes and comprises a surface 2 supported by a rod 10, and intended to carry a blood-collecting bag and to be rocked reciprocatingly 6 on an axle 4 which extends perpendicularly to the surface, by means of a crank 5, 7. The crank may comprise a disc 7 rotatably mounted on a shaft 9 and provided with a wheel 5 which runs on the underside of said carrier surface. The mixer is provided with a weight measuring device 8 which functions to measure the quantity of blood that has flowed into the bag. This apparatus does not exhibit any means for preventing back flow of blood.

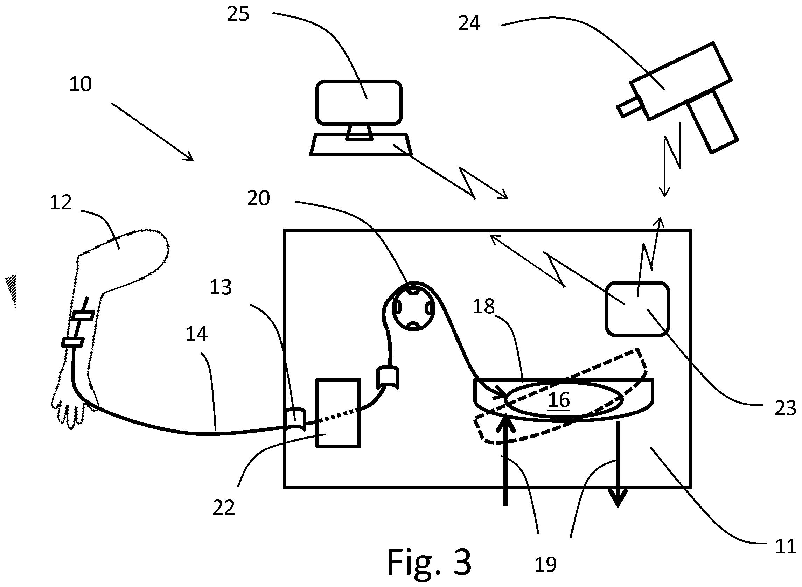

In Fig. 3 the present mixer device/system is schematically illustrated.

The mixer 10 comprises a support structure 11 on which all components are supported, i.e. a tray 18 adapted to hold a blood bag 16, a pump 20, a sensor unit 22 and a transceiver unit 23. There are also provide guide and fastening means, schematically indicated at 13, for securing tubing 14, coupled to the blood bag 16, to the mixer 10.

In Fig. 3 the mixer is shown set up for use. A needle (not shown) is inserted in the arm 12 of the patient, the needle being connected to a tube 14 conducting blood to the colleting system i.e. the blood mixer 10 holding a blood bag 16. The tube 14 is coupled to the collecting bag 16. Tube 14 and bag 16 must be sterile and hence the tube 14 preferably should be continuous all the way from the needle to the blood collecting bag 16. Suitably the blood collecting bag 16 and the tube 14 are delivered as a unit from the manufacturer and in a sterile package that is broken only when it is to be used.

The blood bag 16 is placed in a tray 18, which as is conventional is coupled to a weighing device for weighing the collected blood so as to indicate when the appropriate amount of blood (normally 450 ml) has been collected.

The tray 18 can be tilted at least between two positions, as indicated by a broken line contour, for allowing the blood to mix with agents provided in the bag, such as anticoagulants. Alternatively, the entire mixer can be tiltable. Of course the tilting can implemented with a multi axis system so as to allow tilting in several directions.

A cooling arrangement 19, schematically indicated by arrows only, is implemented in the tray 18 to reduce the temperature of the collected blood to 22°C to improve the quality of the blood. Such cooling can be provided by feeding tempered cooling liquid, such as water, through a channel system in the tray 18. Of course any cooling system known to the skilled man that provides appropriate cooling is usable.

The system 10 comprises a pump 20 for ascertaining a controlled flow of blood from the patient to the blood collecting bag 16. A preferred pump type is a non-intervening pump, i.e. it performs its pumping action by engaging on the outside of the tubing in a kneading action. Thus, it will not affect sterility of the system.

Pumps of this type are often referred to as peristaltic pumps, although other terms are used, such as hose pumps or tube pumps, which differ in construction depending i.e. on the pressure levels prevailing in the systems where the pumps are to be installed.

For the purpose of this application the term "peristaltic pump" is taken to mean any pump type that acts on the exterior surface of tubing and displaces portions of the contents inside the tubing in a desired direction so as to feed it forward.

Furthermore, the present blood mixer is provided with a number of sensors, arranged in a sensor unit schematically indicated at 22. The purpose of the sensors is to increase the safety for the blood donor. To this end there is preferably provided a hemoglobin meter , said hemoglobin meter being configured to continuously measure the hemoglobin value of the collected blood. This is suitably achieved by spectrophotometric principles, using a light source of appropriate wavelengths and a detector for the selected wavelength.

A flow sensor is also preferably provided which is coupled to a regulator configured to regulate the flow of blood by feeding control signals to the pump. The purpose is to prevent pressure build-up in the collecting bag, such that the bag is not damaged, or the patient's veins are undamaged.

Thus, the sensors are configured to:
a) regulate the flow of blood to the collecting bag 16 to prevent pressure build-up in the collecting bag, by monitoring the flow rate and direction,
b) measure the hemoglobin value in the drawn blood using a Hb meter to ensure quality of the blood.

If required, the reading from the flow sensor can be used to activate a stop function if it is registered that blood inadvertently flow backwards and into the patient again in order to prevent blood from flowing from the bag to the donor.

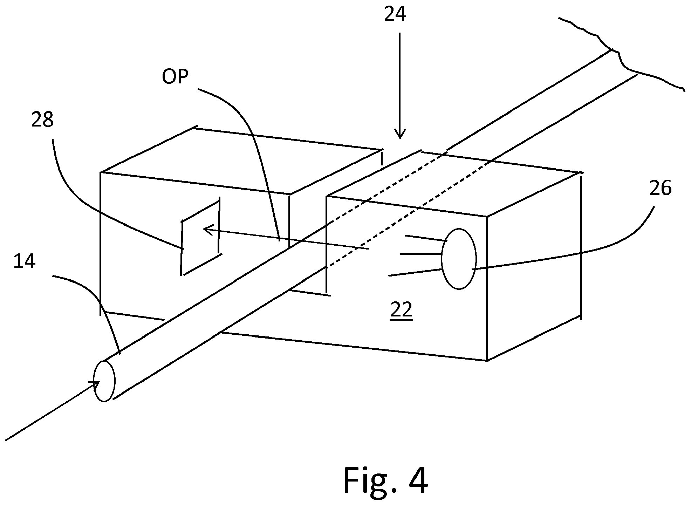

The sensor unit is schematically illustrated in Fig. 4. It is preferably based on optical detection and thereby the blood conducting tube 14 itself is used as a cuvette. The tube is simply placed in a suitable slot 24 in the sensor unit 22 to intervene in the optical pathway (schematically illustrated with an arrow OP) of the detection system. Thus, there is provided a light source 26 (UV, IR or visible depending on what is to be measured) transmitting light of the suitable wavelength across the slot 24, passing the tube with blood inside, and a corresponding detector 28. Reflectivity measurements are also possible.

There can also be provided a pressure sensor for monitoring the pressure inside the tube. Such sensors can be of a piezo-resistive type or any other type that are able to sense the mechanical properties of the tube and correlate them to internal pressure. Any other detection means is of course applicable as long as a reliable flow rate value can be obtained.

The present apparatus comprises a function that prevents the blood to flow from the collecting bag to the donor. This is essential when the blood mixer is placed at an ergonomic level for the staff monitoring the blood collecting procedure since the elevation of the blood mixer may be higher than the position of the blood donor. In cases where a pump that is integrated in the flow path is used, this stop flow function is suitably implemented in the form of a check valve which may be integrated in the pump.

In the preferred embodiments of the present blood mixer the stop flow function will be inherent in that the peristaltic pump type, due its design, will never allow a back flow. Namely, the pump is configured to exert a kneading action on the blood supply tubing. This is achieved in that the pump comprises at least two kneading elements arranged to alternatingly displace the contents of the tube in the direction towards the bag, over one segment of the tubing each, thereby preventing back flow inside the tubing.

In a classic peristaltic pump this kneading and displacing action is performed by rollers often provided on a wheel. The tubing is positioned over at least a part of the circumference of the wheel and suitably clamped against the wheel with a suitable holder, suitably along half the circumference, such that at any time at least one roller will press on the tubing and during a revolution of the wheel the roller will displace liquid inside the tubing over a distance corresponding to half the circumference of the wheel. In this embodiment the pump 20 itself will also act as a fastening means for securing the tubing to the mixer 10.

A welder is used for sealing the blood bag by a clamping and melting action to provide a sterile seal.

The blood mixer may also be provided with a wireless communication device, such as a transceiver, configured to exchange information with a welder 24 and/or a computer 25. The communication can used for recording approved welds of tubing, for traceability purposes, to meet standards of GMP.

All information recorded in the mixer by the sensors is preferably stored in the unit, and is suitably provided as exportable text files although any kind of data format is of course possible. Furthermore, information is suitably used for controlling continued operation of the system. For example a low Hb value could be triggering a system warning to the operator in the form of a question if operation is to be discontinued.

The invention provides for a real-time assessment of the quality of the blood to be extracted by continuously measuring the Hb value of the blood.

The main issue for blood sampling is that the amount of haemoglobin be sufficient in each bag, and preferably the same in all blood bags in a blood bank. Due to the fact that Hb values vary between patients, this is not always the case. The method according to e.g. '553 (mentioned above) will not enable a precise prediction of the amount of haemoglobin in the sampled blood.

By using the apparatus according to the invention, the operator can monitor the accumulation of Hb and terminate the sampling when the exact amount is reached. If the Hb value is high, less blood needs to be collected, and if it is found that the Hb value is too low it is possible to terminate the collection.

Alternatively, the system processor can be programmed to make such decisions automatically.

The invention in a beneficial manner enables a reduction in process steps in blood collection.

## Claims

1. A blood mixer (10) comprising:
a support structure (11) supporting the components of the mixer (10) a tray (18) adapted to house a blood collecting bag having a blood tube (14) attached thereto, and configured to measure the weight of the collected blood in the blood collecting bag (10), and
further comprising a mechanism for causing a repeated tilting of at least the tray (18) in at least one direction
**characterized in that**
a pump (20) is provided on the support structure and configured to draw blood from the donor to a blood collecting bag through said blood tube (14), and **in that**
the pump (20) is configured to exert a kneading action on the blood tube (14) by means of at least two kneading elements arranged to alternatingly displace the contents of the tube in the direction towards the bag, over one segment of the tube each, thereby preventing back flow inside the tube, **in that**
the mixer further comprises a sensor unit which comprises a hemoglobin meter, said hemoglobin meter configured to continuously measure the hemoglobin value of the blood flowing in a tubing secured to the mixer, and **in that**
information recorded by the sensors is stored in a suitable format, preferably as text files.

2. The blood mixer according to claim 1, wherein the sensor unit comprises a flow detector configured to measure flow rate and flow direction of the blood flowing in said tubing.

3. The blood mixer according to claim 2, further comprising a regulator configured to regulate the flow of blood to the collecting bag (10) in response to data from the flow detector.

4. The blood mixer according to claim 1, further comprising a wireless communication device (23) configured to exchange information with a welder (24) and/or a computer (25).

5. The blood mixer according to any preceding claim, further comprising a cooling arrangement (19) configured to cool blood in the blood collecting bag (16).

6. The blood mixer according to any preceding claim, further comprising a tubing fastening and guiding arrangement (13; 20), configured to secure a blood collecting tubing (14) on the mixer (10),

## Patentansprüche

1. Blutmischgerät (10), umfassend:
eine Stützstruktur (11), welche die Komponenten des Mischgeräts (10) stützt,
eine Ablage (18), die dazu adaptiert ist, einen Blutspendesammelbeutel mit einem daran befestigten Blutschlauch (14) aufzunehmen, und dazu konfiguriert ist, das Gewicht des entnommenen Blutes in dem Blutspendesammelbeutel (10) zu messen, und
ferner einen Mechanismus umfassend, der wiederholtes Kippen mindestens der Ablage (18) in mindestens einer Richtung verursacht,
**dadurch gekennzeichnet, dass**
eine Pumpe (20) auf der Stützstruktur vorgesehen ist sowie dazu konfiguriert ist, Blut vom Spender über den besagten Blutschlauch (14) in einen Blutspendesammelbeutel abzunehmen, und dass
die Pumpe (20) dazu konfiguriert ist, mittels mindestens zwei Knetelementen eine Knetwirkung auf den Blutschlauch (14) auszuüben, wobei die Knetelemente so angeordnet sind, dass sie den Inhalt des Schlauches abwechselnd in die Richtung zu dem Beutel hin verdrängen, jeweils über ein Segment des Schlauches, wodurch Rückfluss innerhalb des Schlauches verhindert wird, und dass
das Mischgerät ferner eine Sensoreneinheit umfasst, welche ein Hämoglobinmessgerät umfasst, wobei das besagte Hämoglobinmessgerät dazu konfiguriert ist, den Hämoglobinwert des Blutes, das in einem an dem Mischgerät befestigten Schlauch fließt, kontinuierlich zu messen, und dass die von den Sensoren aufgenommenen Informationen in einem angemessenen Format gespeichert werden, vorzugsweise als Textdateien.

2. Blutmischgerät gemäß Anspruch 1, wobei die Sensoreneinheit einen Strömungsdetektor umfasst, der dazu konfiguriert ist, die Strömungsrate und die Strömungsrichtung des in dem besagten Schlauch fließenden Blutes zu messen.

3. Blutmischgerät gemäß Anspruch 2, ferner einen Regler umfassend, der dazu konfiguriert ist, die Blutströmung zum Sammelbeutel (10) hin in Erwiderung auf Daten von dem Strömungsdetektor zu regeln.

4. Blutmischgerät gemäß Anspruch 1, ferner ein drahtloses Kommunikationsgerät (23) umfassend, das dazu konfiguriert ist, Informationen mit einem Schweißgerät (24) und/oder einem Computer (25) auszutauschen.

5. Blutmischgerät gemäß einem der vorhergehenden Ansprüche, ferner eine Kühlanordnung (19) umfassend, die dazu konfiguriert ist, Blut in dem Blutspendesammelbeutel (16) zu kühlen.

6. Blutmischgerät gemäß einem der vorhergehenden Ansprüche, ferner eine Schlauchbefestigungs- und -führungsanordnung (13; 20) umfassend, die dazu konfiguriert ist, einen Blutspendesammelschlauch (14) an dem Mischgerät (10) zu befestigen.

## Revendications

1. Mélangeur de sang (10) comprenant :
une structure de support (11) supportant les composants du mélangeur (10)
un plateau (18) conçu pour loger un sac de collecte de sang ayant un tube de sang (14) fixé à celui-ci, et configuré pour mesurer le poids du sang collecté dans le sac de collecte de sang (10), et
comprenant en outre un mécanisme pour entraîner une inclinaison répétée au moins du plateau (18) dans au moins une direction
**caractérisé en ce que**
une pompe (20) est prévue sur la structure de support et configurée pour prélever le sang du donneur dans un sac de collecte de sang à travers ledit tube de sang (14) ; et **en ce que**
la pompe (20) est configurée pour exercer une action de malaxage sur le tube de sang (14) au moyen d'au moins deux éléments de malaxage agencés pour déplacer alternativement le contenu du tube dans la direction allant vers le sac, sur un segment de chaque tube, empêchant ainsi le reflux à l'intérieur du tube, **en ce que**
le mélangeur comprend en outre une unité de capteur qui comprend un outil de mesure d'hémoglobine, ledit outil de mesure d'hémoglobine étant configuré pour mesurer de manière continue la valeur d'hémoglobine du sang s'écoulant dans un tubage fixé au mélangeur, et **en ce que**
des informations enregistrées par les capteurs sont stockées dans un format adapté, de préférence sous forme de fichiers texte.

2. Mélangeur de sang selon la revendication 1, dans lequel l'unité de capteur comprend un détecteur d'écoulement configuré pour mesurer le débit et la direction d'écoulement du sang s'écoulant dans ledit tubage.

3. Mélangeur de sang selon la revendication 2, comprenant en outre un régulateur configuré pour réguler l'écoulement de sang dans le sac de collecte (10) en réponse aux données du détecteur d'écoulement.

4. Mélangeur de sang selon la revendication 1, comprenant en outre un dispositif de communication sans fil (23) configuré pour échanger des informations avec un soudeur (24) et/ou un ordinateur (25).

5. Mélangeur de sang selon une quelconque revendication précédente, comprenant en outre un système de refroidissement (19) configuré pour refroidir le sang dans le sac de collecte de sang (16)

6. Mélangeur de sang selon une quelconque revendication précédente, comprenant en outre un système de fixation et de guidage de tubage (13 ; 20), configuré pour fixer un tubage de collecte de sang (14) sur le mélangeur (10).
